# EUROPEAN PATENT SPECIFICATION

(11) **EP 4 429 554 B1**
(45) Date of publication and mention of the grant of the patent: **31.12.2025**
(21) Application number: 22830635.3
(22) Date of filing: 09.11.2022
(51) Int. Cl.: A61B 6/00, A61B 6/42, G01N 23/04

(54) **APPARATUS FOR ENHANCING DIGITAL X-RAY IMAGING**
VORRICHTUNG ZUR VERBESSERUNG DER DIGITALEN RÖNTGENBILDGEBUNG
APPAREIL POUR AMÉLIORER L'IMAGERIE PAR RAYONS X NUMÉRIQUE

(30) Priority: 10.11.2021 US 202163277623 P
(43) Date of publication of application: 18.09.2024
(73) Proprietor: Carestream Health, Inc., Rochester, NY 14608 (US)
(72) Inventor: BOGONI, Luca, Rochester, New York 14608 (US); WANG, Xiaohui, Rochester, New York 14608 (US); JONES, Robert S., Rochester, New York 14608 (US)
(74) Representative: Klang, Alexander H.
(86) International application number: PCT/US2022/049351
(87) International publication number: WO 2023/086351

(56) References cited:
- EP-A1- 3 798 623
- WO-A1-2020/009878
- US-A1- 2018 293 715

## Description

### BACKGROUND OF THE INVENTION

The subject matter disclosed herein relates to methods and apparatus for improving resolution of acquired radiographic images using x-rays.

Structures that are significantly smaller than a digital radiographic detector's interpixel distance might require improved resolution when using standard x-ray imaging techniques. Technology continues improving to reduce the spatial resolution of imaging pixels, however, as pixel size is reduced the amount of incident photons reaching each of the detector's pixels is reduced. In addition, the natural loss of spatial resolution due to digitization may introduce noise, blur, and aliasing.

Multiple radiographic exposures of a patient in clinical practice are less desirable since ionized radiation from an x-ray source is used. In other applications, such as non-destructive testing (NDT), multiple acquisitions are not common practice, as additional time is required. However, there are multiple situations where repeated exposures may not be a significant factor or are employed as part of the usual process/protocol.

WO 2020/009878 A1 discloses improving resolution of an x-ray imaging system so as to obtain super-resolution x-ray images beyond the maximum normal resolution of an x-ray microscope. A method of super-resolution x-ray imaging uses a super-resolving patch classifier. In addition, a method of training the super-resolving patch classifier is disclosed.

EP 3 798 623 A1 discloses using random variation when a reciprocating operation is repeatedly performed in which a stage is moved by (+x, +y) pulses toward an arbitrary position perpendicular to an optical axis of x-rays extending from an x-ray source to an x-ray detector, and then, is moved from there by (-x, -y) pulses. A group of images obtained by moving in parallel to each other is acquired, and an image processing unit finds a deviation between the images, and acquires an input image group in which each of the images has the deviation at a subpixel level. The image processing unit executes a reconstruction processing using the input image group to generate a super-resolution image.

The discussion above is merely provided for general background information and is not intended to be used as an aid in determining the scope of the claimed subject matter.

### BRIEF DESCRIPTION OF THE INVENTION

In accordance with the present invention, a portable radiographic detector assembly for holding and displacing a digital radiographic detector as set forth in claim 1 is provided. Preferred embodiments of the invention are claimed in the dependent claims

The ability of utilizing a super-resolution technique disclosed herein relies on acquiring two or more images. Different embodiments described herein illustrate methods and apparatus in which a pair of images, or more, may be acquired, thus enabling a super resolution technique to be applied. Super resolution techniques have been applied successfully to obtain higher resolutions and sharper images from multiple low-resolution images. Multiple images may be acquired by displacing an image plane by subpixel motion. These images are then combined to reconstruct a higher quality image having increased resolution.

The summary descriptions above are not meant to describe individual separate embodiments whose elements are not interchangeable. In fact, many of the elements described as related to a particular embodiment can be used together with, and possibly interchanged with, elements of other described embodiments. Many changes and modifications may be made within the scope of the present invention.

This brief description of the invention is intended only to provide a brief overview of subject matter disclosed herein according to one or more illustrative embodiments, and does not serve as a guide to interpreting the claims or to define or limit the scope of the invention, which is defined only by the appended claims. This brief description is provided to introduce an illustrative selection of concepts in a simplified form that are further described below in the detailed description. This brief description is not intended to identify key features or essential features of the claimed subject matter, nor is it intended to be used as an aid in determining the scope of the claimed subject matter. The claimed subject matter is not limited to implementations that solve any or all disadvantages noted in the background.

### BRIEF DESCRIPTION OF THE DRAWINGS

So that the manner in which the features of the invention can be understood, a detailed description of the invention may be had by reference to certain embodiments, some of which are illustrated in the accompanying drawings. It is to be noted, however, that the drawings illustrate only certain embodiments of this invention and are therefore not to be considered limiting of its scope, for the scope of the invention encompasses other equally effective embodiments. The drawings below are intended to be drawn neither to any precise scale with respect to relative size, angular relationship, relative position, or timing relationship, nor to any combinational relationship with respect to interchangeability, substitution, or representation of a required implementation., emphasis generally being placed upon illustrating the features of certain embodiments of the invention. In the drawings, like numerals are used to indicate like parts throughout the various views. Thus, for further understanding of the invention, reference can be made to the following detailed description, read in connection with the drawings in which:
FIG. 1 is a schematic diagram of an exemplary radiographic imaging system in one embodiment wherein either the source, detector, object or living body is displaced in the x-y plane at a subpixel distance;
FIG. 2 illustrates: on the left---an imaging step prior to any shifting or displacement of imaging system components, resulting in a first capture of pixel data (1); in the center---another imaging step following a sub-pixel shift, or displacement, of an imaging system component results in a second capture of pixel data (2); on the right: the first captured pixel data (1) and the second captured pixel data (2) are combined using a super-resolution method to achieve an enhanced resolution image (3);
FIGS. 3A-3B are schematic diagrams of a top view and a cross-section, respectively, of a digital detector having a housing that includes a movable, or floating, frame that holds in position an image receptor or digital detector, and controllers to effect *x*-*y* displacement of the frame;
FIG. 4 illustrates the exemplary sub-pixel shifts resulting from the displacements in the captured field of view;
FIG. 5A and 5B illustrate two possible mechanisms to effect displacement of the imaging receptor using a MEMS associated with a flexible element, in FIG. 5A, and using a mechanical drive which may be motorized in FIG. 5B;
FIG. 6 is a schematic perspective view of an exemplary x-ray imaging system; and
FIG. 7 is a schematic diagram of a two dimensional imaging pixel array in a radiographic detector.

### DETAILED DESCRIPTION OF THE INVENTION

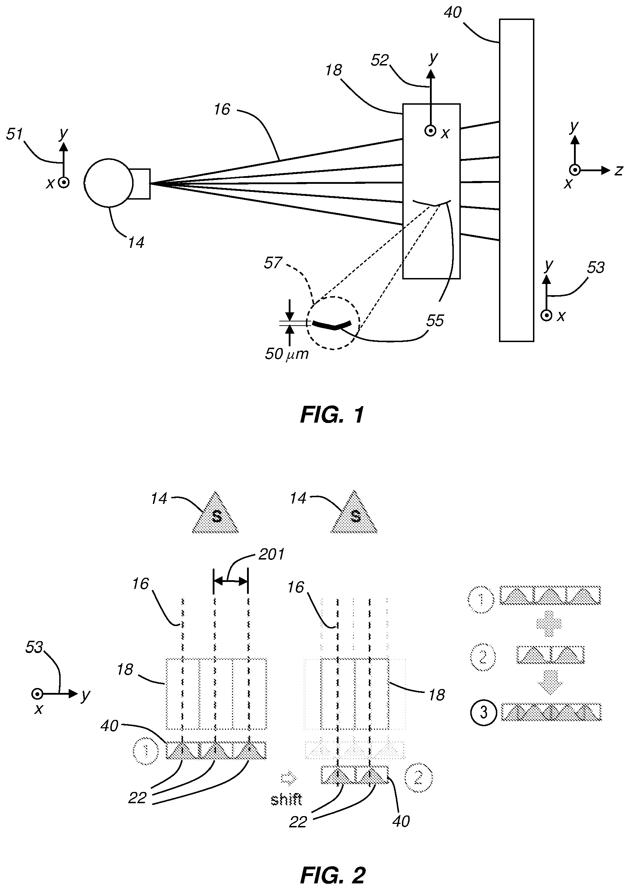

FIG. 1 is a schematic diagram of a radiographic imaging system wherein an object, or a patient, 18 is being exposed to x-rays 16 emitted by a radiation source 14 in order to capture a first radiographic image of the object, or patient, 18 in the digital radiographic detector 40. In one embodiment, after the first radiographic image is captured and stored in the digital detector 40, the source 14 is moved in a preselected direction 51 in the *x-y* plane for a small subpixel distance while the object, or patient, 18 and detector 40 are fixed in position. Then a second radiographic image of the object, or patient, 18 is captured. In another embodiment, after the first radiographic image is captured and stored in the digital detector 40, the object 18 is moved in a preselected direction 52 in the *x-y* plane for a small subpixel distance while the source 14 and detector 40 are fixed in position. Then a second radiographic image of the object, or patient, 18 is captured. In this embodiment, the object 18 might be placed on a movable platform capable of displacing the object, or patient, 18 in the *x-y* plane 52 by a subpixel distance. Such an imaging procedure may be used to characterize, quantify and/or assess the presence of a defect 55 in the object 18, wherein a size of the defect 55 may be smaller than the interpixel distance of the detector 40. As shown in the enlarged image 57 of the defect 55, the defect 55 may have a dimension of about 50 µm. When imaging a patient 18, natural motion of the patient 18 associated with this embodiment can't be adequately controlled, but an additional radiographic image or images of the patient 18, which each contribute image information, may be adequate to increase spatial resolution, decrease noise, and increase contrast when the multiple patient images are combined.

In the context of NDT, in one embodiment, a platform supporting an object 18 to be imaged is configured to be moved in the *x-y* plane 52 on a subpixel scale, i.e., capable of a controlled micro-motion of the platform. The object may be radiographically imaged multiple times, using the imaging system of FIG. 1, after displacing the object 18 by a distance less than the interpixel distance of the detector 40 for each image thus captured, in order to increase the spatial resolution of a final object image generated by combining all of the captured images.

In one embodiment, the digital detector 40 may be placed in a movable frame, as described herein, capable of displacing the detector 40 in the *x-y* plane 53 by a subpixel distance. In this embodiment, the imaging procedure may be used to characterize, quantify and/or assess the presence of a defect 55 in the object 18, wherein a size of the defect 55 may be smaller than the interpixel distance of the detector 40. As shown in the enlarged image 57 of the defect 55, the defect 55 may have a dimension of about 50 µm. As noted, two or more DR images may be acquired in specific circumstances and, while the ability to provide higher resolution images is of high value, the NDT domain enables using multiple exposures without a significant concern of damage to the imaged object 18 that may be caused by radiographic exposure. Super-resolution techniques in digital x-ray imaging can be provided by introducing means for generating image sequences that use subpixel displacements suitable to reconstruct images at increased spatial resolutions.

FIG. 2 illustrates an embodiment of an imaging method using super-resolution. As shown on the left in FIG. 2, x-rays 16 are emitted by an x-ray source 14 through an object 18 toward a digital x-ray detector 40. As these x-rays 16 progress through the object 18 then through a scintillator (not shown) in the digital radiographic detector 40, light energy generated by the x-rays 16 are captured in pixels 22 of the digital detector 40 to form an image of the object 18 therein comprising pixel data (1) which is electronically stored. As shown in the center of FIG. 2, the digital detector 40 is displaced, or shifted, horizontally in the *x-y* plane 53 at a subpixel distance, then another image of the object 18 is captured as pixel data (2) in the digital detector 40 and is electronically stored. The second projected image of the object 18 captured by the image pixels 22 will contain slightly different pixel data (2) as compared with the first captured image containing pixel data (1). As shown on the right in FIG. 2, the two images thus captured can be used to form an image having a higher resolution than either of the original images by combining pixel data (1) and pixel data (2) to form a super resolution image of the object 18 having combined pixel data (3). Using this super-resolution process, an image with enhanced spatial resolution is generated. Whereas super-resolution has been described herein as a means to increase spatial resolution, as a plurality of images are acquired these images may also be used to maintain the same spatial resolution and improve image quality in other aspects, such as increasing sharpness and deblurring areas in the image.

While FIG. 2 illustrates a horizontal shift in the *x-y* plane 53 of the digital detector 40 by about one-half of a pixel width 201, i.e., one-half of the detector's interpixel distance, this relationship can be generalized by stating that super-resolution is accomplished by shifting the imaging plane of the detector 40 by (I + f) pixels, where I = (0,1,2,3), the integer portion of the displacement, and f, the fractional component, is a number > 0 and < 1.0. When values of I > 0 are used, these shifts affect the amount of information digitized along the border area of a captured image and do not bring the benefit of the super-resolution method described herein in the periphery of the image. Thus, in a preferred embodiment, I is most often set to 0. The factional component f is really the key to augment the information and thus increase the spatial resolution. In a preferred embodiment, f is optimally set to 0.5, although other increments may also be useful.

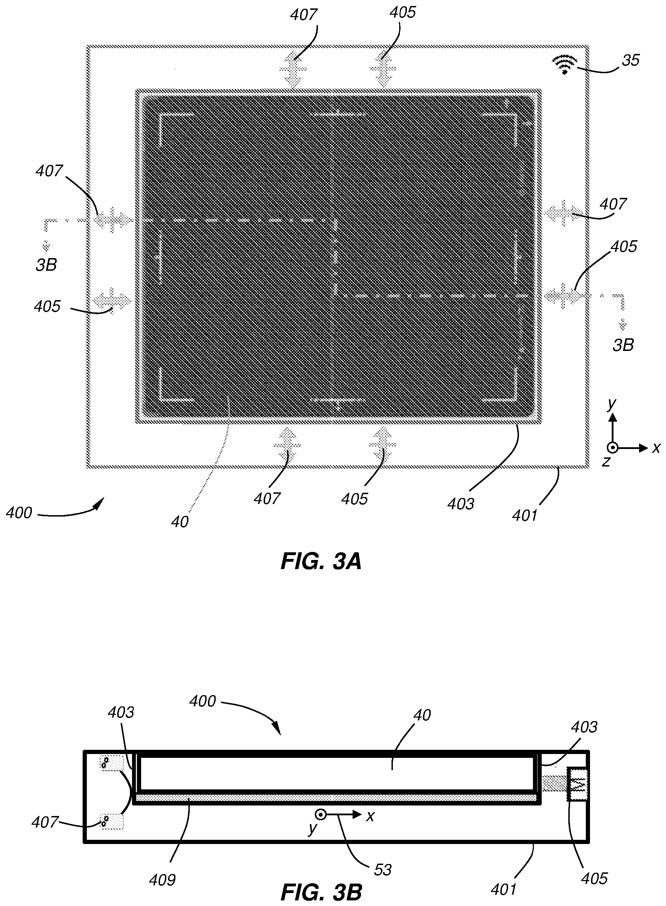

In one embodiment, a detector assembly is configured to apply subpixel movements controllably, systematically and deliberately to a digital detector, or image receptor, 40 so as to enable the acquisition of a plurality of subpixel shifted images and the formation of an enhanced image or a stream of enhanced images. FIGS. 3A-3B illustrate a detector assembly 400 that includes a portable DR detector holder, or housing, 401 having a floating or movable frame 403 that is movable with respect to the housing 401; a wireless communication device 35 (illustrated with a WiFi symbol) to transmit and receive data and instructions such as captured image data and movement commands to activate micro displacement of the frame 403. Alternatively, a cable may be electrically connected to the detector to transmit and receive such data and instructions. A system of micro-controls 405, 407, may be used to drive displacement of the movable frame 403 and the digital detector 40 secured therein relative to the housing 401 of the detector assembly 400 in either one or both of *x* and *y* directions in an *x-y* plane 53 that is generally parallel to a plane of the detector 40 so as to effect subpixel shifts of the detector 40 as described herein. The detector assembly 400 may be fabricated as an integrated nondetachable digital detector apparatus or the detector assembly 400 may be configured to receive a preexisting portable detector 40 that may be removably inserted into the frame 403 of the detector assembly 400 and which may be separately usable as a standalone radiographic digital detector 40 when removed from the housing 401. The frame 403 of the housing 401 may include a pliable yet firm material 409 on one or more inside surfaces thereof for cushioning and securing the digital detector 40 therein.

In one embodiment, an assembly for achieving the subpixel displacements described herein may include a plurality of piezo electronic devices, or micro-electromechanical systems (MEMS) 405, 407, or similar technologies, or a combination thereof, to effect electronically controlled displacement. The MEMS 405, 407, may be attached to the housing 401 and configured to contact two or four sides of the movable frame 403. On each side one or more MEMS 405, 407, may be configured to contact and displace the movable frame 403 portion of the detector assembly 400 as described herein. Upon receiving control instructions, the MEMS 405, 407, may, in response, push the movable frame 403 on a side with which the MEMS 405, 407, is in contact in order to displace the frame 403 in the x-y plane 53 for a selected subpixel distance as indicated by the control instructions. Upon completing a shift in response to control instructions, the detector assembly 400 may be configured to transmit a completion signal indicating the digital detector 40 is in position for a subsequent radiographic image capture procedure. The processing control system, whether on-board the digital detector 40 or external, such as in a connected control system as described herein, may be used to trigger a plurality of exposures resulting in individual radiographic image captures differentiated by subpixel displacements of the detector 40. A program, executed either onboard the digital detector 40 or external to the detector 40, may receive the plurality of acquired radiographic images and generate a combined composite enhanced image.

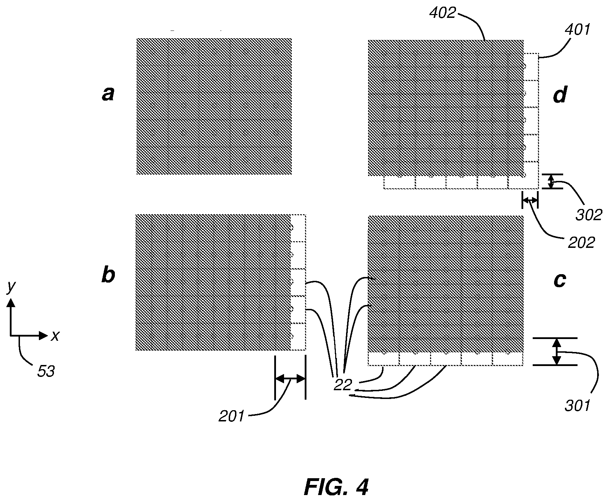

In the context of NDT, certain radiographically imaged structures may be linear in nature and aligned with specific spatial orientations, e.g., parallel lines. In such a situation, a super-resolution strategy may be optimized to capture specific spatial frequencies along a particular direction, such as either in the *x* or *y* dimension of the *x-y* plane, as described herein with respect to FIG. 4. This, in turn, may reduce the number of required sequential image acquisitions needed to achieve spatial resolution and increase image quality.

FIG. 4 illustrates examples of displacement of the imaging plane, or digital detector 40, along the *x-y* plane 53 dimensions. An initial position of the digital detector 40 for capturing a first image of an object, or patient, 18 is represented by the initial detector position 401. A second position of the digital detector 40, displaced by a subpixel distance, for capturing a second image of an object, or patient, 18 is represented by the shaded detector position 402. FIG. 4a represents an exemplary 5×5 array of the detector's image pixels with no shift or displacement of the digital detector 40 as between a first image capture position 401 and a second image capture position 402. This would result in first and second image captures each having equivalent pixel data. FIG. 4b represents the exemplary array of image pixels having a horizontal shift of the digital detector 40 in the *x* dimension of the *x-y* plane 53 as between a first image capture position 401 and a second image capture position 402. The horizontal shift distance 202 is equal to about half of a horizontal interpixel distance 201. This results in first and second image captures having a combined array resolution of 10×5 pixels, with increased resolution in the horizontal *x* dimension. FIG. 4c represents the exemplary array of image pixels having a vertical shift of the digital detector 40 in the *y* dimension of the *x-y* plane 53 as between a first image capture position 401 and a second image capture position 402. The vertical shift distance 302 is equal to about half of a vertical interpixel distance 301. This results in first and second image captures having a combined array resolution of 5×10 pixels, with increased resolution in the vertical *y* dimension. FIG. 4d represents the exemplary array of image pixels having a vertical and horizontal shift of the digital detector 40 in the *x* and *y* dimensions of the *x-y* plane 53 as between a first image capture position 401 and a second image capture position 402. The vertical shift distance 302 is equal to about half of a vertical interpixel distance 301 and the horizontal shift distance 202 is equal to about half of a horizontal interpixel distance 201. This results in first and second image captures having a combined array resolution of 10×10 pixels, with increased resolution in both the horizontal *x* dimension and the vertical *y* dimension. As can be seen in the representations of FIGs. 4b-d, the spatial resolution may be doubled in *x* and/or y dimensions by shifting the imaging plane of the digital detector 40 by half (f = 0.5) of the interpixel distances 201, 301. In practice, the selected displacement may be 0.25 of the interpixel distance 201, 301, of a particular detector 40 (f = 0.25) to further increase the spatial resolution of a combined image. A method using such a displacement distance (f = 0.25) will require more than two additional image acquisitions, i.e., four total image captures-one initial image capture and one for each of three 0.25 displacement steps, and hence three additional x-ray exposures-to be combined as described herein. As described herein, the interpixel distances 201, 301, in the horizontal *x* dimension and in the vertical *y* dimension, respectively, of any particular detector 40 may be equivalent or not. Thus, any gain in resolution may vary with respect to the *x* and *y* dimensions depending on which dimension in the *x-y* plane is selected for the displacement procedure described herein.

It should be understood that the super resolution method described herein may be performed by acquiring a sequence of images where motion, or displacement, may be applied to any of three separate radiographic imaging system components. In one embodiment, wherein the object, or patient, 18 is moved in the *x-y* plane 52, in the context of healthcare, the imaged patient 18 may move naturally such as when acquiring images in a breathing cycle or cardiac cycle. In the context of NDT, the imaged object 18 may be placed on a platform or attached to a device whereby, between image acquisitions, a small, controlled subpixel displacement may be applied in the *x-y* plane 52 (FIG. 1) to the object being imaged. In one embodiment, a small subpixel displacement may be applied in the *x-y* plane 51 (FIG. 1) to the x-ray source 14 between triggering of x-ray emissions therefrom. In one embodiment, a small subpixel motion may be applied to the detector in the *x*-*y* plane 53 (FIG. 1) to introduce small displacements of the detector between image captures. While each of the components discussed herein, source, object/patient, and detector, are described as being shifted or displaced independently from one another, it is understood that there might be a combination of relative movements using two or more of these components to achieve super-resolution.

In the case of x-ray source displacement, the amount of the projective displacement depends on the distance between the source and the detector as well as the position and distance of the object from these components. While it is possible to calibrate the source motion so as to control its displacement relative to pixels in the detector, very small depth and angular misalignments could result in magnified displacement in the imaging plane of the detector. Furthermore, as the imaged object is three-dimensional, possibly with complex internal architecture (e.g., a patient thorax), parallax effects will be induced causing complex effects to be accounted for.

When a super-resolution method such as described herein leverages natural occurring patient body motion, the method may need to account for alignment of anatomical structures. The quality of the intra acquisitions registration will depend on the amount and direction of motion. Super-resolution processes that either use explicit motion models or implicit ones, such as Expectation Minimization or convolutional neural networks, can be used to both align images and integrate the information to achieve a higher spatial resolution, such as shown on the right in FIG. 2. The quality of the super-resolution from multiple images might vary as patient motion may not occur along the projection plane as captured by a detector. If the displacement can be controlled such as by controllably moving the source, object or detector, the information integration and the super-resolution process can be more precise.

In one embodiment, a clinician may be interested in monitoring/imaging a patient's chest during a breathing cycle or visualizing contrast in blood flow as used in serial radiography (fluoroscopy) or C-arm applications. The methods and apparatus disclosed herein may be used to shift a digital detector 40 after each image capture during serial radiography. For example, during a serial radiography imaging procedure that captures and displays images at a rate of eight (8) frames per second, the detector assembly described herein may be used to shift, back and forth, the digital detector 40 eight times per second, once after each image capture during the serial radiography procedure. Each running pair of successively captured images may be combined to enhance the images being displayed during the serial radiographic procedure.

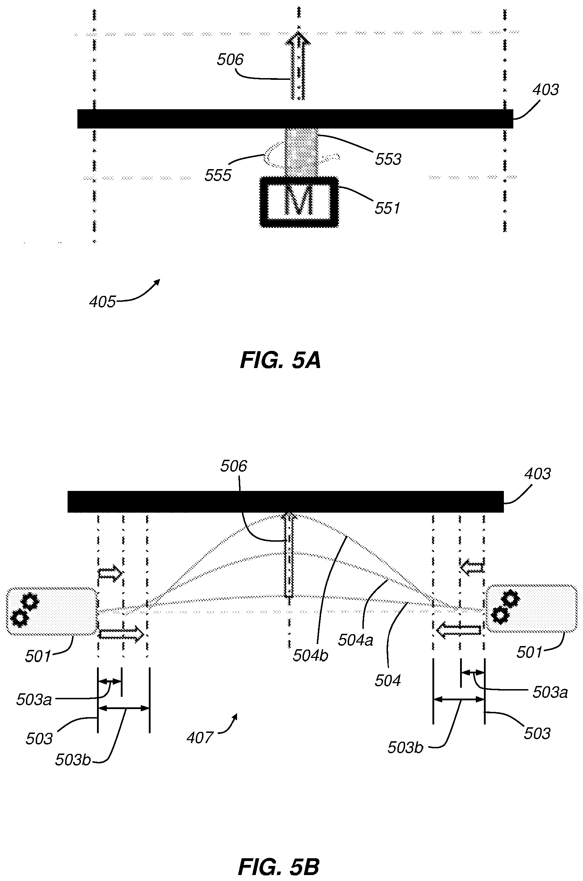

FIGs. 5A-5B are schematic diagrams of exemplary MEMS devices 405, 407, respectively, that may be used to displace the frame 403 and the digital detector 40 therein for a distance less than an interpixel distance of the digital detector 40 as illustrated in FIGs. 3A-3B. FIG. 5A illustrates an exemplary MEMS device 405 attached to the housing 401 (FIGs. 3A-3B), and in contact with the frame 403, that includes a threaded rod 553 and a motor 551 attached thereto for rotating the threaded rod 553 in a clockwise direction 555 to cause the rod to travel in a direction 506 to push the frame 403 in an *x* or *y* direction 506 in the *x-y* plane 53. One or more MEMS devices 405 may be positioned on each of two or four sides of the frame 403 to effect subpixel movement of the movable frame 403 and the digital detector 40 therein in any direction of the *x-y* plane 53. The motor 551 may include a stepper motor, known in the art, that is responsive to digital control instructions communicated thereto. The motor 551 may also be used to rotate the threaded rod 553 in a counterclockwise direction opposite the direction 555 so that the threaded rod 553 may be retracted to allow subpixel movement of the frame 403 in a direction opposite to direction 506. The MEMS devices 405 positioned on other sides of the frame 403 operate in a similar fashion and are coordinated by received control instruction to effect the subpixel displacements of frame 403 as described herein. The threaded rod 553 may be a finely threaded rod having one hundred (100) threads per inch (TPI). Such a threaded rod may be selectively controlled to rotate 1/4 turn in the direction 555 in order to displace the frame 63.5 µm in the direction 506, or the threaded rod 553 may be selectively controlled to rotate 1/8 turn in the direction 555 in order to displace the frame 31.75 µm in the direction 506. If the interpixel distance of the detector 40 is 100 µm, this MEMS device may be used to displace the frame 403 and the digital detector 40 therein for a subpixel distance, as desired. Threaded rods 553 are commercially manufactured having two hundred TPI or more. The thread density of the threaded rod 553 may be selected to provide more than one hundred TPI to provide increased sensitivity when selecting displacement distances for controllably moving the frame 403, as described herein. For example, if a two hundred TPI threaded rod 553 is used, then a 1/8 turn of such a MEMS device 405 would displace the frame 403 for about 15.88 µm. Although subpixel movements of the frame 403 have been described herein, the MEMS device 405 may be selectively used to displace the frame 403 and the digital detector 40 therein for multiple interpixel distances. For example, using the threaded rod 553 having one hundred TPI, one full rotation of the rod 553 would displace the frame 403 and the digital detector 40 therein for about 254 µm; ten full rotations of the rod 553 would displace the frame 403 and the digital detector 40 therein for about 2.54 mm. Any such displacements of the frame 403 and the digital detector 40 may be achieved using the MEMS devices 405, 407 described herein.

FIG. 5B illustrates an exemplary MEMS device 407 attached to the housing 401 (FIGs. 3A-3B), and in contact with the frame 403. MEMS device 407 includes a pair of motion devices 501 each attached to an opposite end of a noncompressible flexible strip 504. The flexible strip 504 may be made of a thin strip of metal, for example, which is thin enough to flex or bend when its ends are pushed together and which returns to its original shape when released. The motion devices 501 act in concert in response to received control instructions to simultaneously move toward each other, thereby bending the flexible strip 504 and causing it to contact and urge the frame 403 in the direction 506 which may be an *x* or *y* direction 506 in the x-*y* plane 53. The motion devices 501 may be instructed to simultaneously move a distance 503a toward each other, from a starting position 503, which causes flexible strip 504 to bend and travel in direction 506 to push frame 403 in an *x* or *y* direction to position 504a. The motion devices 501 may be instructed to simultaneously move a distance 503b toward each other, from a starting position 503 or from position 503a, which causes flexible strip 504 to bend and travel in direction 506 to push frame 403 in an *x* or *y* direction to position 504b. One or more MEMS devices 407 may be positioned on each of two or four sides of the frame 403 to effect subpixel movement of the movable frame 403 and the digital detector 40 therein in any direction of the *x-y* plane 53. The MEMS device 407 may also be used to move the motion devices 501 away from each other so that the flexible strip 504 may be retracted to allow subpixel movement of the frame 403 in a direction opposite to direction 506. The MEMS devices 407 positioned on other sides of the frame 403 operate in a similar fashion and are coordinated by received control instruction to effect the subpixel displacements of frame 403 as described herein.

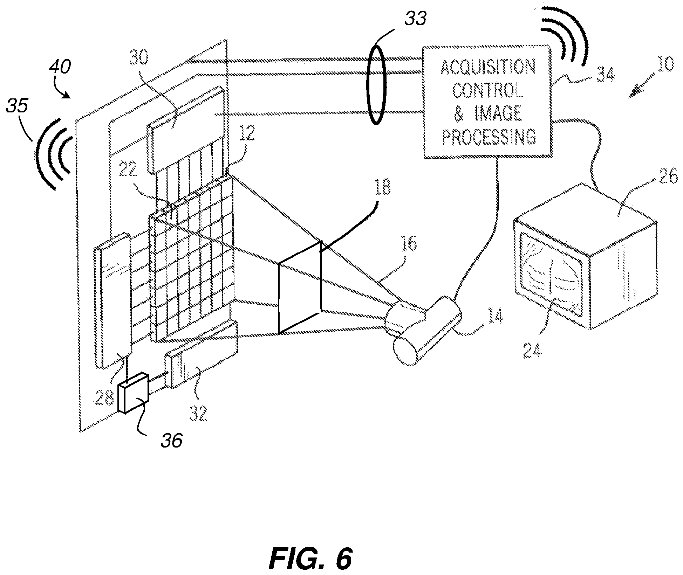

FIG. 6 is a perspective view of an exemplary digital radiographic (DR) imaging system 10, as referenced herein, that may include a generally curved or planar DR detector 40 (shown in a planar embodiment as described herein and without a housing 401 for clarity of description), an x-ray source 14 configured to generate radiographic energy (x-ray radiation), and control station that includes a digital monitor, or electronic display, 26 configured to display images captured by the DR detector 40, and a processing system 34 for controlling operation of the (DR) imaging system 10, according to one embodiment. The DR detector 40 may include a two dimensional array 12 of detector cells 22 (imaging pixels or photosensors), arranged in electronically addressable rows and columns in an *x-y* plane 53. The DR detector 40 may be positioned to receive x-rays 16 passing through an object, or patient, 18 as emitted by the x-ray source 14. As shown in FIG. 6, the radiographic imaging system 10 may use an x-ray source 14 that emits collimated x-rays 16, e.g. an x-ray beam, selectively aimed at and passing through a preselected object, or patient, 18 such that the emitted x-rays 16 fall on an imaging region, i.e., an image receptor formed as a two-dimensional array of imaging pixels 12, of the DR detector 40. The x-ray beam 16 may be attenuated by varying degrees along its plurality of rays according to the structure, e.g., varying thickness, of the object, or patient, 18, which attenuated x-rays are detected by the array 12 of imaging pixels 22. The curved or planar DR detector 40 may be positioned, as much as possible, in a perpendicular relation to a central ray of the plurality of rays 16 emitted by the x-ray source 14. The array 12 of individual imaging pixels 22 may be electronically addressed (scanned) by their position according to column and row. As used herein, the terms "column" and "row" refer to the vertical and horizontal arrangement of the photosensor cells 22 in an *x-y* plane and, for clarity of description, it will be assumed that the rows extend horizontally and the columns extend vertically. However, the orientation of the columns and rows is arbitrary and does not limit the scope of any embodiments disclosed herein. Each individual imaging pixel 12 may be scanned by readout circuitry 28, 30, described herein, to determine a stored voltage level generated therein by incoming x-ray radiation. The voltage level stored in each imaging pixel 12 may be read out by the circuitry 28, 30, and stored electronically as a digitized numerical value. As is well known, an A/D converter may be used to convert the stored voltage level in each pixel 12 into a digital value. A higher numerical value may be understood to represent a greater amount of x-ray energy absorbed by an individual imaging pixel 12 during an imaging procedure of an object, or patient, 18.

In one exemplary embodiment, the rows of photosensitive cells 22 may be scanned one or more at a time by electronic scanning circuit 28 so that the exposure data from the array 12 may be transmitted to electronic read-out circuit 30. Each photosensitive cell 22 may independently store a charge proportional to an intensity, or energy level, of the attenuated radiographic radiation, or x-rays, received and absorbed in the cell. Thus, each photosensitive cell, when read-out, provides information defining a pixel of a radiographic image 24, e.g. a brightness level or an amount of energy absorbed by the pixel, that may be digitally decoded by image processing electronics 34 and transmitted to be displayed by the digital monitor 26 for viewing by a user. An electronic bias circuit 32 is electrically connected to the two-dimensional detector array 12 to provide a bias voltage to each of the photosensitive cells 22.

Each of the bias circuit 32, the scanning circuit 28, and the read-out circuit 30, may communicate with an acquisition control and image processing unit 34 over a connected cable 33 (wired), or the DR detector 40 and the acquisition control and image processing unit 34 may each be equipped with a wireless transmitter/receiver to transmit radiographic image data wirelessly 35 to the acquisition control and image processing unit 34. The acquisition control and image processing unit 34 may include a processor and electronic memory (not shown) to control operations of the DR detector 40, and the imaging system 10 overall, as described herein, including control of circuits 28, 30, and 32, for example, by use of programmed instructions transmitted to the detector 40, and to store and process image data. The acquisition control and image processing unit 34 may also be used to control activation of the x-ray source 14 during a radiographic exposure, controlling an x-ray tube electric current magnitude, and thus the fluence of x-rays in x-ray beam 16, and/or the x-ray tube voltage, and thus the energy level of the x-rays in x-ray beam 16. A portion, or all, of the acquisition control and image processing unit 34 functions may reside in the detector 40 in an on-board processing system 36 which may include a processor and electronic memory to control operations of the DR detector 40 as described herein, including control of circuits 28, 30, and 32, and transmitter/receiver 35 by use of programmed instructions, and to store and process image data similar to the functions of standalone acquisition control and image processing system 34. The on-board processing system 36 may receive instructions transmitted from control and image processing system 34 in order to control operations of the MEMS devices 405, 407, as described herein. The processing system 36 may perform image acquisition and image disposition functions as described herein. The processing system 36 may control image transmission and image processing and image correction on board the detector 40 based on instructions or other commands transmitted from the acquisition control and image processing unit 34, and transmit corrected digital image data therefrom, and may transmit status information related to completed subpixel displacements of the frame 403 so that subsequent image capture procedures may be initiated.

The processing system 36 may convey control signals to the MEMS devices 405, 407, to controllably displace the frame 403 and the digital detector 40 therein according to instructions received from image processing unit 34. The processing system 36 may store predetermined instructions to automatically shift the frame 403 and the digital detector 40 therein according to a preselected shift distance. Such preselected shift distances may be stored in processing system 36 as absolute values, e.g., 50 µm or another absolute distance value, or they may be stored as f = 0.25 or f = 0.5 values, which may be programmably applied by processing system 36 corresponding to the pixel resolution of the detector 40. The processing system 36 may also cooperate with an image processing unit 34 which may be a part of a serial radiography (fluoroscopy) system. The processing system 36 may signal the serial radiography system each time that the frame 403 is shifted (back and forth) to synchronize each serial image capture with the programmed back and forth shift of the digital detector 40.

With regard to a direct detection embodiment of DR detector 40, the photosensitive cells 22 may each include a sensing element sensitive to x-rays, i.e. it absorbs x-rays and generates an amount of charge carriers in proportion to a magnitude of the absorbed x-ray energy. A switching element may be configured to be selectively activated to read out the charge level of a corresponding x-ray sensing element. With regard to an indirect detection embodiment of DR detector 40, photosensitive cells 22 may each include a sensing element sensitive to light rays in the visible spectrum, i.e. it absorbs light rays and generates an amount of charge carriers in proportion to a magnitude of the absorbed light energy, and a switching element that is selectively activated to read the charge level of the corresponding sensing element. A scintillator, or wavelength converter, (not shown) may be disposed over the light sensitive sensing elements to convert incident x-ray radiographic energy to visible light energy. Thus, in the embodiments disclosed herein, it should be noted that the DR detector 40 may include an indirect or direct type of DR detector.

Examples of sensing elements used in sensing array 12 include various types of photoelectric conversion devices (e.g., photosensors) such as photodiodes (P-N or PIN diodes), photo-capacitors (MIS), photo-transistors or photoconductors. Examples of switching elements used for signal read-out include a-Si TFTs, oxide TFTs, MOS transistors, bipolar transistors and other p-n junction components.

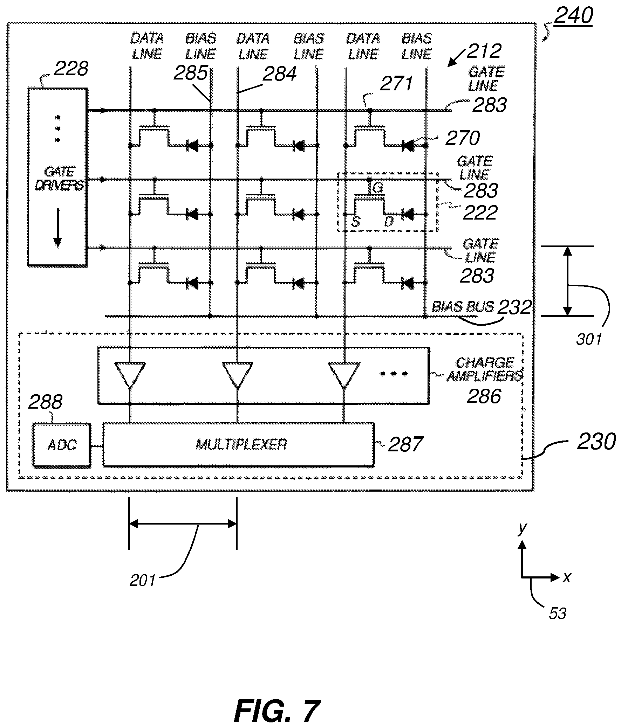

FIG. 7 is a schematic diagram 240 of a portion of a two-dimensional array 12 for a DR detector 40. The array of photosensor cells 212, whose operation may be consistent with the photosensor array 12 described above, may include a number of hydrogenated amorphous silicon (a-Si:H) n-i-p photodiodes 270 and thin film transistors (TFTs) 271 formed as field effect transistors ( FETs) each having gate (G), source (S), and drain (D) terminals. In embodiments of DR detector 40 disclosed herein, the two-dimensional array of photosensor cells 12 may be formed in a device layer that abuts adjacent layers of the DR detector structure, which adjacent layers may include a rigid glass layer or a flexible polyimide layer or a layer including carbon fiber without any adjacent rigid layers. A plurality of gate driver circuits 228 may be electrically connected to a plurality of gate lines 283 which control a voltage applied to the gates of TFTs 271, a plurality of readout circuits 230 may be electrically connected to data lines 284, and a plurality of bias lines 285 may be electrically connected to a bias line bus or a variable bias reference voltage line 232 which controls a voltage applied to the photodiodes 270. Charge amplifiers 286 may be electrically connected to the data lines 284 to receive signals therefrom. Outputs from the charge amplifiers 286 may be electrically connected to a multiplexer 287, such as an analog multiplexer, then to an analog-to-digital converter (ADC) 288, or they may be directly connected to the ADC, to stream out the digital radiographic image data at desired rates. In one embodiment, the schematic diagram of FIG. 7 may represent a portion of a DR detector 40 such as an a-Si:H based indirect flat panel, curved panel, or flexible panel imager.

Incident x-rays, or x-ray photons, 16 are converted to optical photons, or light rays, by a scintillator, which light rays are subsequently converted to electron-hole pairs, or charges, upon impacting the a-Si:H n-i-p photodiodes 270. In one embodiment, an exemplary detector cell 222, which may be equivalently referred to herein as a pixel, may include a photodiode 270 having its anode electrically connected to a bias line 285 and its cathode electrically connected to the drain (D) of TFT 271. The bias reference voltage line 232 can control a bias voltage of the photodiodes 270 at each of the detector cells 222. The charge capacity of each of the photodiodes 270 is a function of its bias voltage and its capacitance. In general, a reverse bias voltage, e.g. a negative voltage, may be applied to the bias lines 285 to create an electric field (and hence a depletion region) across the pn junction of each of the photodiodes 270 to enhance its collection efficiency for the charges generated by incident light rays. The image signal represented by the array of photosensor cells 212 may be integrated by the photodiodes while their associated TFTs 271 are held in a non-conducting (off) state, for example, by maintaining the gate lines 283 at a negative voltage via the gate driver circuits 228. The photosensor cell array 212 may be read out by sequentially switching rows of the TFTs 271 to a conducting (on) state by means of the gate driver circuits 228. When a row of the pixels 22 is switched to a conducting state, for example by applying a positive voltage to the corresponding gate line 283, collected charge from the photodiode in those pixels may be transferred along data lines 284 and integrated by the external charge amplifier circuits 286. The row may then be switched back to a non-conducting state, and the process is repeated for each row until the entire array of photosensor cells 212 has been read out. The integrated signal outputs are transferred from the external charge amplifiers 286 to an analog-to-digital converter (ADC) 288 using a parallel-to-serial converter, such as multiplexer 287, which together comprise read-out circuit 230.

This digital image information may be subsequently stored immediately as controlled by an on-board processing system 36, or it may be image processed on-board, or it may be processed by image processing system 34 to yield a digital image which may then be digitally stored and immediately displayed on monitor 26, or it may be displayed at a later time by accessing a digital electronic memory containing the stored image. The flat panel DR detector 40 having an imaging array as described with reference to FIG. 7 is capable of both single-shot (e.g., static, radiographic) and continuous (e.g., fluoroscopic) image acquisition.

As will be appreciated by one skilled in the art, aspects of the present invention may be embodied as a system, method, or computer program product. Accordingly, aspects of the present invention may take the form of an entirely hardware embodiment, an entirely software embodiment (including firmware, resident software, micro-code, etc.), or an embodiment combining software and hardware aspects that may all generally be referred to herein as a "service," "circuit," "circuitry," "module," and/or "system." Furthermore, aspects of the present invention may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

Program code and/or executable instructions embodied on a computer readable medium may be transmitted using any appropriate medium, including but not limited to wireless, wireline, optical fiber cable, RF, etc., or any suitable combination of the foregoing.

Computer program code for carrying out operations for aspects of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer (device), partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described herein with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art.

## Claims

1. A portable radiographic detector assembly (400) for holding and displacing a digital radiographic detector (40), the assembly (400) comprising:
a moveable frame (403) having an opening configured to receive and secure the digital radiographic detector (40); and
micromechanical devices (405, 407) attached to the frame (403) and configured for displacing the frame (403) and the digital radiographic detector (40), the micromechanical devices (405, 407) controllable to displace the digital radiographic detector (40) at a distance of a non-integer number of the digital radiographic detector's (40) interpixel distance;
wherein the micromechanical devices (405, 407) are controllable to displace the digital radiographic detector (40) less than the interpixel distance of the digital radiographic detector (40);
wherein the detector assembly (400) further comprises a housing (401), wherein the micromechanical devices (405, 407) are further attached to the housing (401), and wherein the frame (403) is configured to be displaced relative to the housing (401);
wherein the detector assembly (400) includes a wireless data transmission/receiving device (35) configured to receive displacement instructions for displacing the frame (403) for a selected distance corresponding to the displacement instructions; and
wherein the micromechanical devices (405, 407) each comprise a rotatable threaded rod (553).

2. The detector assembly (400) of claim 1, wherein the detector assembly (400) includes electronic memory for storing the non-integer number of the digital radiographic detector's (40) interpixel distance.

3. The detector assembly (400) of claim 1, further comprising a processing system (36) for synchronizing the displacement of the digital radiographic detector (40) by the micromechanical devices (405, 407) with each image capture during a serial radiography procedure.

4. The detector assembly (400) of claim 1, wherein the micromechanical devices (405, 407) are configurable to displace the digital radiographic detector (40) in each of two perpendicular directions.

## Patentansprüche

1. Eine tragbare Röntgendetektoranordnung (400) zum Halten und Verschieben eines digitalen Röntgendetektors (40), wobei die Anordnung (400) Folgendes aufweist:
einen bewegbaren Rahmen (403) mit einer Öffnung, die so konfiguriert ist, dass sie den digitalen Röntgendetektor (40) aufnimmt und befestigt; und
mikromechanische Vorrichtungen (405, 407), die an dem Rahmen (403) angebracht und zum Verschieben des Rahmens (403) und des digitalen Röntgendetektors (40) konfiguriert sind, wobei die mikromechanischen Vorrichtungen (405, 407) so steuerbar sind, dass sie den digitalen Röntgendetektor (40) um einen Abstand verschieben, der einer nicht ganzzahligen Anzahl des Interpixelabstands des digitalen Röntgendetektors (40) entspricht;
wobei die mikromechanischen Vorrichtungen (405, 407) so steuerbar sind, dass sie den digitalen Röntgendetektor (40) um weniger als den Interpixelabstand des digitalen Röntgendetektors (40) verschieben;
wobei die Detektoranordnung (400) ferner ein Gehäuse (401) aufweist, wobei die mikromechanischen Vorrichtungen (405, 407) ferner an dem Gehäuse (401) angebracht sind und wobei der Rahmen (403) so konfiguriert ist, dass er relativ zu dem Gehäuse (401) verschoben werden kann;
wobei die Detektoranordnung (400) eine drahtlose Datenübertragungs- /Empfangsvorrichtung (35) beinhaltet, die so konfiguriert ist, dass sie Verschiebungsanweisungen zum Verschieben des Rahmens (403) um einen ausgewählten Abstand entsprechend den Verschiebungsanweisungen empfängt; und
wobei die mikromechanischen Vorrichtungen (405, 407) jeweils eine drehbare Gewindestange (553) aufweisen.

2. Die Detektoranordnung (400) nach Anspruch 1, wobei die Detektoranordnung (400) einen elektronischen Speicher zum Speichern der nicht ganzzahligen Anzahl des Interpixelabstands des digitalen Röntgendetektors (40) aufweist.

3. Die Detektoranordnung (400) nach Anspruch 1, die ferner ein Verarbeitungssystem (36) zum Synchronisieren der Verschiebung des digitalen Röntgendetektors (40) durch die mikromechanischen Vorrichtungen (405, 407) mit jeder Bildaufnahme während eines seriellen Röntgenverfahrens aufweist.

4. Die Detektoranordnung (400) nach Anspruch 1, wobei die mikromechanischen Vorrichtungen (405, 407) so konfigurierbar sind, dass sie den digitalen Röntgendetektor (40) in jeder von zwei senkrechten Richtungen verschieben.

## Revendications

1. Ensemble détecteur radiographique portable (400) destiné à maintenir et à déplacer un détecteur radiographique numérique (40), l'ensemble (400) comprenant :
un châssis mobile (403) comportant une ouverture configurée pour recevoir et fixer le détecteur radiographique numérique (40) ; et
des dispositifs micromécaniques (405, 407) fixés au châssis (403) et configurés pour déplacer le châssis (403) et le détecteur radiographique numérique (40), les dispositifs micromécaniques (405, 407) pouvant être commandés pour déplacer le détecteur radiographique numérique (40) d'une distance correspondant à un nombre non entier de la distance interpixel du détecteur radiographique numérique (40) ;
dans lequel les dispositifs micromécaniques (405, 407) peuvent être commandés pour déplacer le détecteur radiographique numérique (40) d'une distance inférieure à la distance interpixel du détecteur radiographique numérique (40) ;
dans lequel l'ensemble de détecteur (400) comprend en outre un boîtier (401), dans lequel les dispositifs micromécaniques (405, 407) sont en outre fixés au boîtier (401), et dans lequel le châssis (403) est configuré pour être déplacé par rapport au boîtier (401) ;
dans lequel l'ensemble détecteur (400) comprend un dispositif de transmission/réception de données sans fil (35) configuré pour recevoir des instructions de déplacement afin de déplacer le châssis (403) sur une distance sélectionnée correspondant aux instructions de déplacement ; et
dans lequel les dispositifs micromécaniques (405, 407) comprennent chacun une tige filetée rotative (553).

2. L'ensemble détecteur (400) selon la revendication 1, dans lequel l'ensemble détecteur (400) comprend une mémoire électronique pour stocker le nombre non entier de la distance interpixel du détecteur radiographique numérique (40).

3. L'ensemble détecteur (400) selon la revendication 1, comprenant en outre un système de traitement (36) pour synchroniser le déplacement du détecteur radiographique numérique (40) par les dispositifs micromécaniques (405, 407) avec chaque capture d'image pendant une procédure de radiographie en série.

4. L'ensemble détecteur (400) selon la revendication 1, dans lequel les dispositifs micromécaniques (405, 407) sont configurables pour déplacer le détecteur radiographique numérique (40) dans chacune des deux directions perpendiculaires.
